# NEW EUROPEAN PATENT SPECIFICATION

(11) **EP 2 686 006 B2**
(45) Date of publication and mention of the opposition decision: **19.02.2020**
(45) Mention of the grant of the patent: 14.12.2016
(21) Application number: 12711464.3
(22) Date of filing: 16.03.2012
(51) Int. Cl.: A61K 38/44, A61K 31/522, A61P 25/32

(54) **COMPOSITION COMPRISING DIAMINE OXIDASE FOR THE PREVENTION OF HANGOVER SYMPTOMS**
ZUSAMMENSETZUNGEN ENTHALTEND DIAMINOXIDASE ZUR VORBEUGUNG DES "HANGOVERS"
COMPOSITION PERMETTANT D'EVITER LES SYMPTOMES LIES A LA "GUEULE DE BOIS" CONTENANT DE LA DIAMINE OXIDASE

(30) Priority: 18.03.2011 ES 201130380 P
(43) Date of publication of application: 22.01.2014
(73) Proprietor: Dr Healthcare España, S. L., 08017 Barcelona (ES)
(72) Inventor: DUELO RIU, Carlos, 08017 Barcelona (ES); DUELO RIU, Juan José, 08017 Barcelona (ES)
(74) Representative: Ponti & Partners, S.L.P
(86) International application number: PCT/IB2012/051275
(87) International publication number: WO 2012/127391

(56) References cited:
- WO-A1-2006/003213
- US-A- 4 703 045
- SHIMODA ET AL: "Investigation of the mechanism of alcohol-induced bronchial asthma", JOURNAL OF ALLERGY AND CLINICAL IMMUNOLOGY, MOSBY, INC, US, vol. 97, no. 1, 1 January 1996 (1996-01-01), pages 74-84, XP005111447, ISSN: 0091-6749, DOI: 10.1016/S0091-6749(96)70285-3
- LINNEBERG A ET AL: "Genetic determinants of both ethanol and acetaldehyde metabolism influence alcohol hypersensitivity and drinking behaviour among Scandinavians", CLINICAL & EXPERIMENTAL ALLERGY, WILEY INTERSCIENCE, vol. 40, no. 1, 1 January 2010 (2010-01-01), pages 123-130, XP009158868, ISSN: 1365-2222, DOI: 10.1111/J.1365-2222.2009.03398.X
- Haeberle M: "Supplementation of diamine-oxidas - a new approach in the treatment of histamine intolerance", ACTA DERMATO-VENEREOLOGICA, vol. 87, no. 5, 1 September 2007 (2007-09-01), pages 459-480, XP055418118,
- Brunello Wüthrich: "Nigrane-Attacken, Tropfnase, Bauchgrimmen", Wein Goermet, 2007, pages 119-120,
- DAOSIN
- SWIFT, R: "Alcohol hangover", Alcohol Health & Research World, vol. 22, no. 1, 1998, pages 54-60,

## Description

### FIELD OF THE INVENTION

The present invention refers to a composition comprising diamine oxidase (DAO) and caffeine to block the effects of histamine release caused by consumption of alcoholic drinks and therefore to prevent hangover symptoms.

### BACKGROUND TO THE INVENTION

The effects of alcohol on the organism depend on a series of individual factors and on the environment, as well as on the quantity that has been drunk. The absorption of alcohol, or ethanol, occurs when the drink enters the body by the mouth, passes to the oesophagus and reaches the stomach where it is diluted by gastric juices. The speed with which alcohol passes from the stomach to the intestine to mix with the blood flow and to produce its effects is determined by:
- Type of drink and amount of alcohol that it contains (the higher the alcohol concentration, the more rapid its absorption).
- Speed of drinking (the higher the drinking speed, the more rapidly it is absorbed).
- Presence of food in the stomach, especially fats (the presence of food delays alcohol absorption).
- Body weight and gender (women and thin people absorb alcohol more rapidly).
- Mood, emotional and general health (tiredness, depression and poor health increase absorption speed).
- Previous consumption experience.

Alcohol reaches the Central Nervous System (CNS), including the brain, through the blood. The effects, both subjective (the way the drinker feels that changes their mood and perception of things), and objective (the behaviour they exhibit) start to appear almost immediately.

Alcohol or ethanol is a CNS depressant, an anaesthetic; it is not a stimulant. In small quantities, drinks containing alcohol seem to stimulate because they inhibit the cerebral functions related to learning, judgement and control. This initial disinhibition and euphoria, which can appear after drinking small quantities, have made people believe wrongly that alcoholic drinks are stimulants.

Hangover is the result of intoxication of the organism, caused by the ingestion of an excessive dose of alcohol. Hangover is the appearance of a series of symptoms on the day after having drunk alcohol excessively and can become worse if the person smokes excessively.

The organism protects itself from intoxication and secretes enzymes that metabolise and excrete the toxins. However, when the ingestion of alcohol is excessive the capacity of the organism to metabolise it is less and the symptoms of hangover appear.

Some studies have shown that hangover is due to the metabolic processes of the liver, the diuretic effects of alcohol and the reduction in blood sugar.

When alcohol reaches the blood (between 30 and 90 minutes after being ingested), the level of sugars in the circulation is reduced, which causes a feeling of weakness and physical exhaustion. This is because alcohol accelerates the transformation of glycogen (a substance that is responsible for storing sugar in the liver) to glucose, which is excreted more rapidly.

Another action of alcohol is that it inhibits vasopressin, which is a hormone secreted by the suprarenal gland. This hormone is responsible for maintaining liquid balance in the body, stimulating the kidneys to reabsorb water from the urine. If vasopressin function is inhibited, the kidneys start to eliminate more water than is ingested and causes the organism to seek water from other organs. This causes the meninges (membranes covering the brain) to lose water and therefore produces a headache.

Headache, common in all people suffering from hangover, results from dilation of the blood vessels due to the effect of some vasodilator substances (such as histamine) in the organism.

Histamine [2-(4-imidazolyl)ethylamine] is an important mediator of many biological processes including inflammation, secretion of gastric acid, neuromodulation and the regulation of the immune function. Due to its potent pharmacological activity, even at very low concentration, it is necessary to regulate the synthesis, transport, storage, release and degradation of histamine very carefully in order to avoid undesirable reactions. High concentrations of free histamine in the circulation lead to undesirable effects such as headache, blocked nose or rhinorrhoea, obstruction of respiratory pathways, tachycardia, gastric and intestinal pains, eyelid swelling, cutaneous erythema, reduction of arterial pressure, bronchospasms, etc.

Histamine is produced by human beings and stored in an inactive form in the metachromatic granules of the mast cells and basophilic leukocytes, where it is available for immediate release. The highest concentrations of histamine are measured in the lungs. After release, histamine is an extraordinary powerful mediator of a large number of physiological and pathophysiological processes, frequently mediated by interaction with the cytokines.

Histamine can also enter the human body from the outside as it is generated by microbiological action in the course of processing foods and, consequently, is present in substantial quantities in many fermented foods and drinks such as wine, champagne and a large proportion of alcoholic drinks.

Therefore after ingesting significant amounts of alcoholic drinks, a significant increase in circulating histamine is produced.

The main route of inactivation of ingested histamine is oxidative deamination of the primary amino group, catalysed by diamine oxidase (DAO) to produce imidazole acetaldehyde.

The main function of DAO is to prevent histamine ingested with food from reaching the blood circulation from the intestine.

In addition to histamine, DAO can degrade other biogenic amines such as, for example, putrescine, spermidine and cadaverine. It has a molecular weight of approximately 182 kDa and a carbohydrate ratio of 11 %. It belongs to the copper-containing amine oxidase class that catalyses oxidative deamination of primary amines to give aldehydes, ammonia and hydrogen peroxide. DAO uses molecular oxygen for the oxidative deamination of histamine to imidazole acetaldehyde, ammonia and hydrogen peroxide. 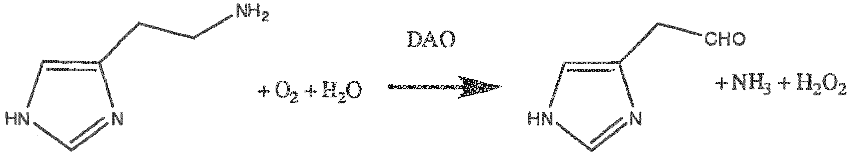 Histamine Imidazole acetaldehyde

DAO is mainly found in the small intestine, liver, kidneys and blood leukocytes. The level of DAO in the blood of pregnant women is approximately 500 to 1000 times higher than of non-pregnant women, as DAO in pregnant women is additionally formed in the placenta. Histamine is continuously produced in humans and is excreted via the intestine, being degraded on passing through intestinal mucosa by the DAO found there.

DAO is a sensitive enzyme that can be inhibited by different substances such as other biogenic amines, alcohol and by its degradation product acetaldehyde, as well as by various medicinal drugs.

Thus, after significant consumption of alcoholic drinks, a double negative effect is produced for the human organism. Firstly, the alcohol consumed inhibits DAO activity of degrading histamine, and secondly, the amount of histamine in the blood circulation increases with the majority of alcoholic drinks. As a consequence of this double effect, non-degraded histamine passes into the circulation causing the symptoms of hangover.

In this situation, preventative administration of supplementary amounts of DAO has the effect of degrading excess histamine derived from consumption of alcoholic drinks, compensating for the inhibition of DAO caused by the alcohol consumption.

Patent EP 132674 described a procedure for the enzymatic separation of free amines in foods containing high amine content such as chocolate, cheese, especially mature, salami, wine and yeast extracts by the use of amine oxidase enzymes, particularly DAO, obtained from *Aspergillus niger,* in the presence of molecular oxygen. The presence of these free amines in certain foods is thought to cause migraines.

Patent US 4725540 described a procedure for preparing DAO from a microorganism that makes it such as *Candida crusei* or a bacterium that produces lactic acid in a nutrient medium so that the DAO produced is capable of degrading histamine at a pH of between neutral and approximately 4.

Patent application WO 02/43745 from 2001 described the systematic use of DAO of plant origin for the treatment of diseases mediated by histamine, particularly for the treatment of allergies in general and anaphylactic reactions in particular. There are also pharmaceutical compositions comprising DAO as the active ingredient, including corresponding dosages and administration protocols. The DAO used originates from plants. No mention has been made of the possible use of DAO compositions for the prevention of the symptoms of hangover.

Patent application WO 2006003213 of 2005 referred to pharmaceutical compositions for the treatment of histamine-induced diseases, comprising DAO of animal origin where the composition is presented in a form protected against gastric acid for oral or perioral administration. The compositions are particularly directed for the treatment of urticaria, atopic dermatitis and scombrotoxic poisoning. This patent application favours the use of DAO of non-plant origin because it is argued that this form has the advantage that there are no plant allergens present to have a negative effect on the administration of the DAO, as allergens essentially promote the release of endogenous histamine. The DAO used is preferably obtained from pig kidneys or by recombinant techniques. No mention has been made of the possible use of DAO compositions for the prevention of the symptoms of hangover.

### Definitions

**"DAO"** is the abbreviation used for the enzyme diamine oxidase responsible for catalysing the oxidative deamination of the primary amine group of histamine to give imidazole acetaldehyde. It is responsible for the main route of histamine inactivation.

**"Hangover"** is a general discomfort suffered on waking by a person who has drunk alcohol to excess and consists of:
- Headache, produced by dilation of blood vessels caused by the accumulation of histamine
- Red eyes
- Occasional memory loss
- Vomiting
- Possible flatulence
- Intense thirst, which originates as a response of the body to dehydration caused by the alcohol
- Abdominal and muscular pain, which results in a feeling of weakness
- In some cases, diarrhoea

**"Non-plant origin"** means DAO that is not obtained from plants but from animal organisms or from other non-plant organisms. Thus all DAO isolated from living things falls under this definition.

**"Plant origin"** means all DAO obtained from plant organisms.

**"Biotechnological origin"** means all recombinant DAO prepared from cell cultures or in non-vegetable organisms of any type where the DNA for DAO has been isolated.

**"Prevention"** in the context of the present invention is avoiding the appearance of one or more of the symptoms related to hangover.

### SUMMARY OF THE INVENTION

The problem solved by the present invention is that of preventing the undesirable effects of hangover produced as a consequence of the ingestion of alcoholic drinks.

The first aspect of the present invention is the composition comprising DAO associated with caffeine for use in the prevention of the symptoms associated with hangover.

The second aspect of the present invention is oral compositions comprising DAO containing caffeine, in the form of tablets, capsules and sachets.

The third aspect of the present invention is oral compositions comprising DAO prepared from free DAO, in powder, lyophilised powder, microcapsules, nanocapsules or liposomes containing DAO and caffeine.

The fourth aspect of the present invention are oral compositions comprising DAO prepared from free DAO, in powder, lyophilised powder, microcapsules, nanocapsules or gastro-protected liposomes containing DAO and also caffeine.

### DETAILED DESCRIPTION OF THE INVENTION

The present invention refers to a composition comprising diamine oxidase and caffeine for oral administration for use in the prevention of hangover symptoms produced by the consumption of alcoholic drinks.

An embodiment of the first aspect is a composition comprising DAO and caffeine for use in the prevention of the symptoms associated with hangover.

The DAO used in the present invention can be either of biotechnological origin or extracted from animals or plants.

If the DAO used is of non-plant origin, it is preferably in the form of a lyophilised powder. If the DAO used is or plant origin, it may also be in liquid form.

The different compositions comprising DAO and caffeine for use in the prevention of the symptoms associated with hangover, are in the form of tablets, capsules or sachets containing DAO in free form, in powder, lyophilised powder, microcapsules, nanocapsules or liposomes of DAO with gastric protection.

The different compositions comprising DAO also contain caffeine to strengthen the effects of preventing the symptoms associated with hangover.

Caffeine is an alkaloid of the xanthine group that has vasoconstriction capacity. Histamine produces vasodilation and this vasodilation produces pain. Caffeine contributes to vasoconstriction and therefore to alleviate the pain.

The DAO content in compositions of the present invention is between 0.1 and 50 mg per dose, preferably between 2 and 20 mg.

The caffeine content in compositions of the present invention is between 1 and 100 mg per dose, preferably between 5 and 50 mg.

Compositions comprising DAO and caffeine for use in the prevention of the symptoms associated with hangover must be taken between 1 hour and 15 minutes before the start of consuming alcoholic drinks, preferably ½ hour before the start of consuming alcoholic drinks.

The compositions comprising DAO and caffeine of the present invention directly affects the level of histamine in blood and therefore the undesirable effects produced by the ingestion of alcoholic drinks, known as hangover symptoms, but does not influence the level of alcohol in blood.

The compositions of the present invention are prepared from free DAO, in powder, lyophilised powder, microcapsules, nanocapsules or liposomes of DAO that have an enteric coating layer that protects the DAO from gastric acidity, so that the different forms can be packaged directly in sachets or put into a capsule or compressed to give tablets. The enteric coating layer that coats the different forms rapidly disintegrates or dissolves in a neutral or alkaline medium.

In the case of microgranules, the cores can be inert cores based on sugar or similar over which the DAO is applied or these cores can already contain DAO mixed with other excipients. These excipients can be binders, surfactants, filling materials, disintegrators, alkaline additives or other pharmaceutically acceptable ingredients either alone or in a mixture. The binders can be a cellulose-like binder such as hydroxypropyl methylcellulose, hydroxypropyl cellulose and sodium carboxymethyl cellulose, polyvinylpyrrolidone, sugars, starches and other pharmaceutically acceptable substances with cohesive properties. Suitable surfactants can be from the groups of ionic or non-ionic acceptable surfactants such as, for example, sodium lauryl sulphate.

Alternatively, DAO can be mixed with alkaline compounds and additionally mixed with constituents suitable for formulation in a core material. These core materials can be produced by extrusion/spheronization or by compression using different processing equipment.

DAO can also be mixed with other pharmaceutically acceptable alkaline substances such as salts of phosphoric acid and sodium, potassium, calcium, magnesium and aluminium; carbonic acid, citric acid or other suitably weak organic or inorganic acids; a co-precipitate of aluminium hydroxide/sodium bicarbonate; substances normally used in antacid preparations such as aluminium, calcium and magnesium hydroxides; magnesium oxide or compound substances such as Al₂O₃.6MgO.CO₂.12H₂O, (Mg₆Al₂(OH)₁₆CO₃.4H₂O, MgO.Al₂O₃.2SiO₂.nH₂O or similar compounds; pH buffering substances such as tris(hydroxymethyl)aminomethane, basic amino acids and their salts or other pharmaceutically acceptable pH buffering substances.

The enteric coating layers can contain pharmaceutically acceptable plasticizers to obtain the desired mechanical, flexibility and hardness properties. These plasticizers can be, for example, triacetin, citric acid esters, phthalic acid esters, cetyl alcohol, polyethylene glycols, polysorbates or other plasticizers.

The present invention also refers to a treatment method comprising administration of an oral composition comprising DAO and caffeine, according to any of the embodiments of the present invention, to a patient who presents symptoms of hangover produced by consuming alcoholic drinks or who is at risk from suffering from them, in a therapeutically effective amount.

### EXAMPLES

### Reference Example 1

Tablets of DAO were prepared from microgranules containing 4 % DAO in accordance with the following formula:

| | |
|---|---|
| DAO | 4 mg |
| Mannitol | 40 mg |
| Microcrystalline cellulose | 25 mg |
| Hydroxypropyl cellulose | 10 mg |
| Maize starch | 10 mg |
| Citric acid | 6 mg |

The microgranules were coated with hydroxypropyl methylcellulose.

To make the tablets, the DAO microgranules were compressed with microcrystalline cellulose and sodium stearyl fumarate.

### Example 2

Tablets of DAO were prepared from microgranules containing 4 % DAO and 10 % caffeine in accordance with the following formula:

| | |
|---|---|
| DAO | 4 mg |
| Caffeine | 10 mg |
| Mannitol | 35 mg |
| Microcrystalline cellulose | 15 mg |
| Hydroxypropyl cellulose | 10 mg |
| Hydroxypropyl methylcellulose | 10 mg |
| Ascorbic acid | 6 mg |

The microgranules were coated with a methacrylic acid copolymer.

To make the tablets, the DAO microgranules were compressed with microcrystalline cellulose and magnesium stearate.

### Reference Example 3

DAO sachets were prepared containing 100 or 150 mg of DAO microgranules prepared according to the first part of reference example 1.

### Example 4

DAO and caffeine sachets were prepared containing 100 or 150 mg of DAO microgranules prepared according to the first part of example 2.

### Reference Example 5

DAO capsules were prepared containing 100 or 150 mg of DAO microgranules prepared according to the first part of reference example 1, filling the soft gelatine capsules with the microgranules.

### Example 6

DAO and caffeine capsules were prepared containing 100 or 150 mg of DAO microgranules prepared according to the first part of example 2, filling the soft gelatine capsules with the microgranules.

### Example 7

Oral compositions containing DAO, either alone or associated with caffeine, the object of the present invention, were tested in a total of 52 subjects (40 men and 12 women with ages between 21 and 65 years), as outpatients.

To check the relation between the symptoms of hangover and the histamine level in blood, histamine levels were analysed before and after taking the trial alcoholic drinks. Normal histamine values for blood were considered to be between 2 and 20 micrograms histamine per decilitre of blood. Elevated histamine values for blood were considered to be over 20 micrograms histamine per decilitre of blood.

The following tables show the results in relation to the absence of symptoms of hangover such as: sickness, cephalea, general discomfort, tiredness, gastrointestinal disorders, etc., after preventative administrations of DAO compositions of the present invention prior to consuming alcoholic drinks.

The consumption of alcoholic drinks that was followed in the protocol of the present trial consisted in taking: 3 glasses of wine, one cognac and two alcoholic drinks (whisky, gin, vodka, rum, or similar).

The study was carried out by dividing the group of volunteer subjects into two subgroups of 26 subjects in each. The subjects in the first group were administered compositions of DAO ½ hour before starting to consume alcoholic drinks. The subjects in the second group did not take DAO compositions before starting to consume alcoholic drinks.

**Table 1: Comparative results of the symptoms of hangover between subjects taking DAO tablets, of Reference Example 1, and those not taking DAO.**

| Hangover symptoms | Subjects taking DAO: 26 | Subjects not taking DAO: 26 | |
|---|---|---|---|
| Cephalea | 4 of 26 | 24 of 26 | |
| Gastro-intestinal disorders | 2 of 26 | 20 of 26 | |
| Vomiting | 2 of 26 | 18 of 26 | |
| Histamine level in blood | 2-20 micrograms/0.1 L | >> | 20 micrograms/0.1 L |

**Table 2: Comparative results of the symptoms of hangover between subjects taking DAO and caffeine tablets, of Example 2, and those not taking DAO and caffeine.**

| Hangover symptoms | Subjects taking DAO and caffeine: 26 | Subjects not taking DAO and caffeine: 26 | |
|---|---|---|---|
| Cephalea | 3 of 26 | 23 of 26 | |
| Gastro-intestinal disorders | 1 of 26 | 19 of 26 | |
| Vomiting | 0 of 26 | 19 of 26 | |
| Histamine level in blood | 2-20 micrograms/0.1 L | >> | 20 micrograms/0.1 L |

## Claims

1. Composition comprising diamine oxidase (DAO) and caffeine for use in the prevention of hangover symptoms produced by the consumption of alcoholic drinks.

2. Composition in accordance with claim 1, wherein the composition is administered orally in the form of tablets, capsules or sachets.

3. Composition in accordance with claims 1 and 2, wherein the DAO in the composition is administered at a dose of between 0.1 and 50 mg.

4. Composition in accordance with claims 1 to 3, wherein the caffeine is administered at a dose of between 1 and 100 mg.

5. Composition in accordance with claims 1 to 4, wherein the dose forms have gastric protection.

6. Composition in accordance with claims 1 to 5, wherein the DAO is used in free form, in powder, lyophilised powder, microcapsules, nanocapsules or liposomes.

7. Composition in accordance with claims 1 to 6, wherein the composition is administered between 1 hour and 15 minutes before starting to consume alcoholic drinks.

## Patentansprüche

1. Zusammensetzung mit Diaminoxidase (DAO) und Koffein für die Verwendung bei der Verhinderung von Kater-Symptomen, die durch den Konsum alkoholischer Getränke verursacht werden.

2. Zusammensetzung für die Verwendung gemäß Anspruch 1, wobei die Zusammensetzung in der Form von Tabletten, Kapseln oder Portionsbeuteln oral verabreicht wird.

3. Zusammensetzung für die Verwendung gemäß Anspruch 1 und 2, bei der die DAO in der Zusammensetzung mit einer Dosierung von zwischen 0,1 und 50 mg verabreicht wird.

4. Zusammensetzung für eine Verwendung gemäß einem der Ansprüche 1 bis 3, bei der das Koffein mit einer Dosis von zwischen 1 und 100 mg verabreicht wird.

5. Zusammensetzung für die Verwendung gemäß einem der Ansprüche 1 bis 4, bei der die Dosierungsformen Magenschutz aufweisen.

6. Zusammensetzung für die Verwendung gemäß einem der Ansprüche 1 bis 5, bei der die DAO in freier Form, in Pulver, lyophilisiertem Pulver, Mikrokapseln, Nanokapseln oder Liposomen verwendet wird.

7. Zusammensetzung für die Verwendung gemäß einem der Ansprüche 1 bis 6, wobei die Zusammensetzung zwischen einer Stunde und 15 Minuten vor dem Beginn des Konsums alkoholischer Getränke verabreicht wird.

## Revendications

1. Composition comprenant de la diamine oxydase (DAO) et de la caféine devant être utilisée dans la prévention des symptômes de la xylostomiase produits par la consommation de boissons alcoolisées.

2. Composition devant être utilisée selon la revendication 1, dans laquelle la composition est administrée par voie orale sous la forme de comprimés, capsules ou sachets.

3. Composition devant être utilisée conformément aux revendications 1 et 2, dans laquelle la DAO de la composition est administrée à une dose comprise entre 0,1 et 50 mg.

4. Composition devant être utilisée conformément aux revendications 1 à 3, dans laquelle la caféine est administrée à une dose comprise entre 1 et 100 mg.

5. Composition devant être utilisée conformément aux revendications 1 à 4, dans laquelle les formes de présentation des doses contiennent une protection gastrique.

6. Composition devant être utilisée conformément aux revendications 1 à 5, dans laquelle la DAO est utilisée sous forme libre, en poudre, poudre lyophilisée, microcapsules, nanocapsules ou liposomes.

7. Composition devant être utilisée conformément aux revendications 1 à 6, dans laquelle la composition est administrée entre 1 heure et 15 minutes avant de commencer à consommer des boissons alcoolisées.
